# EUROPEAN PATENT APPLICATION

(11) **EP 2 457 602 A1**
(43) Date of publication of application: **30.05.2012**
(21) Application number: 10192607.9
(22) Date of filing: 25.11.2010
(51) Int. Cl.: A61M 5/142, A61M 5/168, A61M 39/22, F16K 5/06, F16K 11/00, F16K 17/00, F16K 24/02, A61M 5/48

(54) **Infusion pump having dosing unit with safety valve**

(71) Applicant: F. Hoffmann-La Roche AG, 4070 Basel (CH); Roche Diagnostics GmbH, 68305 Mannheim (DE)
(72) Inventor: Niklaus, Hanspeter, 4853, Riken (CH); Haueter, Ulrich, 3506, Grosshöchstetten (CH); Teutsch, David, 3054, Schüpfen (CH); Kühni, Florian, 3084 Wabern (CH)
(74) Representative: Rentsch Partner AG

(57) **Abstract**

A valve (4) for an infusion pump device (2) comprises a pump connection (41 ) intended to be fluidly connected to a piston pump (11,12,13), an infusion site connection (42) intended to be fluidly connected to an infusion set (31,33), and a reservoir connection (43) intended to be fluidly connected to a liquid medicament reservoir (21 ). The valve (4) can be in a first state (I), where the pump connection (41 ) is fluidly connected to the infusion site connection (42), and the reservoir connection (43) is sealingly closed, and in a second state (II), where the pump connection (41 ) is fluidly connected to the reservoir connection (43), and the infusion site connection (42) is sealingly closed. The valve (4) comprises one or more conduits, grooves, and/or recesses (441) vented to environment (46) that are arranged in such a way that no path can exist within the valve between the infusion site connection (42) and the reservoir connection (43) that would not cross at least one of said conduits, grooves, and/or recesses (441 ), thereby establishing a drain, in case that one of the sealings of the connections would leak. In this way, leakage between the reservoir and infusion site connection is prevented in case of valve malfunction.

## Description

### Field of the Invention

The invention relates to valves and dosing units for infusion pump devices and to methods for conveying liquid medicament in an infusion pump device.

### State of the art

Devices for the automated release of liquid medicaments are normally used with patients who have a continuous and in the course of the day varying need of a liquid medicine which can be administered by infusion. Specific applications are, for example, certain pain therapies, cancer therapies and the treatment of diabetes mellitus, in which computer controlled infusion pump devices are used. Such devices are particularly useful for ambulatory therapy, and are generally carried attached on or near the body of a patient. The medicine reservoir often comprises medicine supply sufficient for one or several days. The liquid medicament is supplied to the patient's body from the medicine reservoir through an infusion cannula or an injection needle.

Ambulatory infusion pump devices are typically of the syringe driver type, where the liquid medicament to be administered to the patient is stored in a cylindrical glass cartridge or ampoule acting as the liquid medicament reservoir, and is conveyed to the body of the patient by displacing a piston within the cylinder. An example of such an infusion pump device is disclosed for example in WO 01/83008 A1, and is schematically depicted in Figure 1(a). A cylinder 11 of the dosing unit 1 comprises the complete reservoir 21 of liquid medicament of the infusion pump device 2. An outlet 17 is fluidly connected 32 to an infusion tubing 31, which on its other end is fluidly connected to an infusion site interface 33 attached to the body of a patient 9. A piston head 12 arranged in the cylinder is unidirectionally displaced along the cylinder axis by an drive system 14 via a piston shaft or threaded spindle 13. The drive system is controlled by a control unit 22.

A number of drawbacks of such a design are known in the art. In particular, such pump devices have a limited precision, because they involve pumping very small volumes, typically in the nanolitre range, out of a cartridge having an overall volume in the range of millilitre, typically e.g. 3 ml. Thus, to achieve a precise dosing of the liquid medicament, it is necessary to very precisely displace the piston. Already small deviations can lead to over dosing or under dosing. Furthermore the forces needed to actuate the piston are comparably high due to the friction between the walls of the glass cartridge and the sealing of the piston. This leads to demanding requirements for the drive system and the mechanical parts involved, as well as the control unit of the pump. As a consequence such infusion pump devices are expensive.

Another problem is the lower limit of the length of such an infusion pump device. The complete supply of liquid medicament has to be stored in the cartridge acting as the pump cylinder. The cross-sectional area of the piston has to be below a certain limit, for precision reasons and in order to limit the device thickness, which is known to be a particularly critical dimension with respect to comfort and discreetness during application. The minimum overall length of the device is then essentially given by the resulting minimum length of the cylinder, which is detrimental to the provision of compact infusion pumps.

Particularly in self-administration of medicaments, for example insulin, the patients using the medicament in question and administering it themselves by means of an infusion pump are increasingly emphasizing convenience and discretion, which restricts the acceptable size and weight of such devices. Particular the overall length, width and thickness should be as small as possible, in order not be evident through clothing and to be carried as comfortable as possible.

Alternative approaches have been proposed, in which a separate dosing unit is provided downstream from the liquid medicament reservoir. Since the primary reservoir does not have to fulfil additional functions, its dimensions can be optimized in view of the compactness of the infusion pump device. Such a dosing unit can comprise for example a micro membrane pump or a micro piston pump, especially designed for precise metering of small volumes. A piston pump with smaller dimensions retrieves liquid medicament from a larger primary reservoir, e.g. a collapsible reservoir, and conveys the liquid medicament in a precise manner to the injection site on the body of the patient.

When filled, the cylinder of the piston pump acts as a secondary reservoir, holding a restricted amount of liquid medicament. When the cylinder is empty, the piston pump retrieves new liquid medicament from the primary reservoir. Such pumps are generally full-stroke pumps, where the cavity of a membrane pump or the cylinder of a piston pump is always completely emptied. Hence the inner volume of the pump must correspond to the smallest volume increment that may have to be delivered, typically in the nanoliter range.

While several designs for such dosing units are known in the art, they are rather complex, expensive and critical with respect to large scale manufacture since they integrate a number of functional components, in particular metering components and valves and are frequently made from materials which are costly and/or critical in production and processing, such as silicon.

WO 2007/077255 A2 discloses a simpler infusion pump device, where check valves, realized by flexible wings of a plunger arranged in the cylinder of the dosing unit, ensure the correct flow of the liquid medicament during the refilling mode and the pumping mode. To ensure user safety, such a design requires a cost intensive drive system, since any uncontrolled activation of the drive system due to a malfunction would inevitably lead to a an overdosing event.

The liquid medicaments that are administered by liquid infusion pump devices are generally highly effective. The accuracy of the dosing unit is therefore of utmost importance, to avoid any potentially hazardous dosing errors. Such accuracy can be ensured by an appropriate design of the various parts of an infusion pump device, as well as by using high quality components.

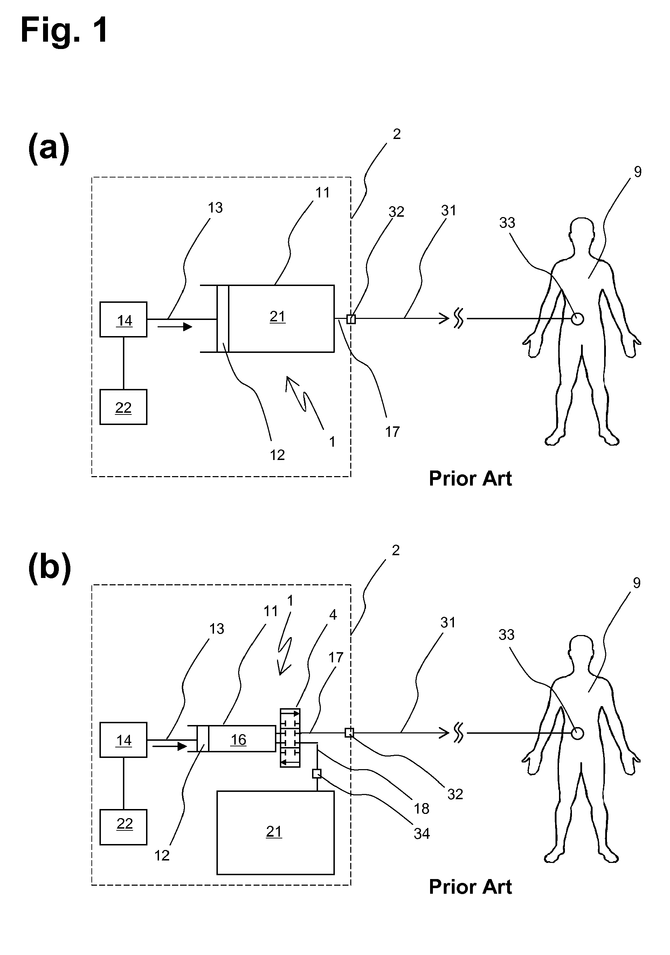

As an additional level of safety, the dosing unit can be construed in a way that even the a complete malfunction of one component, for example the drive system, as unlikely as it may be, cannot lead to an overdosing hazard. For that purpose a second component of the device has to intervene. Infusion pump devices with such a design are known from the prior art. In one variant of such an infusion pump device, a 4/3 or 3/3 way valve is arranged at a front end of the cylinder of a dosing unit, as schematically shown in Figure 1 (b).

A piston 12, 13 arranged in the cylinder of the dosing unit 1 can be bidirectionally displaced along the cylinder axis by a drive system. In a first state of the valve 4, an inlet conduit 18 fluidly connected to the primary reservoir 21 is fluidly connected to the cylinder, and an outlet conduit 17 fluidly connected to the infusing tubing is disconnected from the dosing unit. This state of the valve is applied during the refill mode, when the dosing unit retracts the piston and sucks liquid medicament from the primary reservoir 21 into the cylinder.

In a second state of the valve, as it is shown in Figure 1(b), both the inlet 18 and the outlet 17 are disconnected from the dosing unit 1. This blocked state of the valve is applied when, for example, the infusion tubing has to be temporarily disconnected from the infusion pump device.

In a third state of the valve, the cylinder of the dosing unit is fluidly connected to the outlet conduit 17, thereby establishing a fluid connection to the body of the patient 9. The inlet conduit 18 is disconnected from the dosing unit. This third valve state is applied during the pumping mode, when liquid medicament is conveyed from the secondary reservoir 16 in the cylinder of the dosing unit to the subcutaneous tissue of the patient.

During application, the dosing unit is in the third state most of the time. For refilling, it is in the first state for a time span in a range of some seconds to maximal some minutes, depending on design parameters such as the cylinder volume and the displacement speed of the piston. The second state is passed only when switching between the first state and the third state. In typical embodiments, the system is such that only the first state and the third state can be moved into in a defined way.

A piston pump based dosing unit with an advantageous variant of such a valve system is known from EP 1970677 A1 of the applicants. The valve, a 3/3 or 4/3 way valve, is realized as a rotatable cylinder head acting as a valve member, which interacts with a fixed cylinder tube, acting as the valve seat. Alternatively, a rotatable cylinder can act as the valve member, mounted in a fixed valve seat. A particularly advantageous embodiment of the latter variant is disclosed in EP 2163273 A1 of the applicants, where the actuating means of the piston indirectly actuate also the valve member by rotating the cylinder, which is frictionally connected to the piston. The disclosure of these two documents is hereby included by reference in its entirety.

A device in accordance with this design generally provides a high safety level. However, in the generally unlikely but well possible case of both valves showing a leakage, caused, e.g., by a problem in the manufacture, the cylinder may be bridged, resulting in a direct fluidic connection between the inlet and the outlet.

Since the health of a patient is of primary importance and be protected by all means, there is accordingly a need to further improve the safety level of all infusion pump devices known from the prior art.

### Objects of the Invention

It is the overall objective of this invention to provide an advantageous valve for use in an infusion pump device with increased safety, which especially prevents an uncontrolled flow to the patient in case of a valve defect as described in the previous section. Further favourable properties of embodiments of devices in accordance with the present disclosure will be come visible as the description proceeds.

Such a valve further should allow a precise dosing of liquid medicament. The valve should be reliable, and should be producible with high quality at low costs in a large-scale manufacture.

Another object of the invention is to provide a dosing unit with such a valve, and an infusion pump device with such a valve.

A further object of the invention is to provide a advantageous method for safely conveying liquid medicament in an infusion pump device.

These and other objects are achieved by a valve for an infusion pump device, a dosing unit for an infusion pump device, an infusion pump device with, and a method for conveying liquid medicament in an infusion pump device, according to the independent claims. Advantageous embodiments and variants are given in the dependent claims.

### Summary of the invention

The present invention can comprise one or more of the features recited in the claims, and/or one or more of the following features and combinations thereof.

The basic principle of a dosing unit according to the invention is the insertion of an additional mode between the refilling mode, where the pump cylinder is fluidly connected with the primary reservoir holding the liquid medicament supply, and the pump mode, where the pump cylinder is fluidly connected to the infusion set, or the patient respectively. In said additional mode the pump cylinder is temporarily connected to environment. This allows the equalisation of a positive or negative pressure difference between the cylinder and the environment; because the additional mode is passed each time the dosing unit switches between refilling mode and pumping mode. Each switching is associated with pressure equalization. This prevents the unintentional and uncontrolled administration of medicament due to overpressure in the cylinder, as well as the retraction of fluid from the infusion set into the cylinder. A specially designed valve arranged between the pump cylinder, the primary reservoir, and the infusion set realizes this additional venting mode.

The environment can by the inner volume of a device housing, particularly a hermetically sealed housing. The environment can also be the surrounding atmosphere.

The design is such that any liquid that may otherwise directly flow from the primary reservoir to the infusion set via a shortcut, thus circumventing the pump cylinder, has to pass one or more conduits, grooves, and/or recesses connected to environment. Accordingly said liquid leaves the fluid system via these one or more conduits, grooves, and/or recesses toward the environment. Such a situation may result, as explained before, due to a defect or malfunctioning valve.

A basic embodiment of a valve according to the invention for an infusion pump device comprises a pump connection intended to be fluidly connected to a piston pump, a infusion site connection intended to be fluidly connected to an infusion set, and a reservoir connection intended to be fluidly connected to a liquid medicament reservoir. The valve can be in a first state, where the pump connection s fluidly connected to the infusion site connection, and the reservoir connection is sealingly closed, and in a second state, where the pump connection is fluidly connected to the reservoir connection, and the infusion site connection is sealingly closed. The valve comprises one or more conduits, grooves, and/or recesses connected to environment that are arranged in such a way that no path exists within the valve between the infusion site connection and the reservoir connection, independently if such a path is closed during normal operation of the valve, that does not cross at least one of said conduits, grooves, and/or recesses, thereby establishing a drain to environment.

Said environment can for example be inner volume of a compartment of a device housing, particularly of a hermetically sealed device housing. In such a case the environmental pressure is the specific pressure inside the device housing. The environment can also be the surrounding atmosphere. In that case the one or more conduits, grooves, and/or recesses are connected atmospheric pressure.

The conduits, grooves, and/or recesses should provide a flow path that has a fluidic resistance toward environment that is low, particularly as compared to a closed valve path.

In an advantageous embodiment of a valve according to the invention the valve can be in a third state, where the pump connection is fluidly connected to environment, and the other connections are sealingly closed. The valve is designed in such a way that the valve has to switch to the third state before it can to the first state and/or the second state. Advantageously the one or more conduits, grooves, and/or recesses serve as the fluidic connection to environment in the third state.

Since the conduit is passed for each switching, an over pressure that may be present in the primary reservoir, caused, e.g., by high temperature and/or mechanical stress that is exerted onto the primary reservoir, is equalized each time the dosing unit is switched between the refilling mode and the pump mode.

The valve should advantageously be designed such that the switching process does not lead to the shifting even of small amounts liquid within the fluid system. This measure further increases the accuracy of a dosing unit according to the invention. This can favourably be achieved via a rotational rather than a linear valve motion.

Advantageously the valve according to the invention is designed such that during switching between the different states the valve goes to an intermediary state, where all connections are disconnected.

The valve can comprise a valve seat and a valve member that are rotatable to each other. In such an embodiment it is particularly advantageous if the valve seat comprises at least one passage conduit that is connected to the pump connection, and if the valve member comprises a conduit fluidly connected to the infusion site connection and a conduit fluidly connected to the reservoir connection, wherein the fluid connection between a passage conduit and the connection conduits is established when an opening o the passage conduit overlaps with an opening of a connection conduit. In an even more advantageous embodiment such valve according to the invention comprises one or more venting conduits fluidly connectable to a passage conduit.

The valve can comprise means provided on the valve seat and the valve member that limit the rotational displacement in regard to each other, wherein one limit of rotational displacement corresponds to the first state of the valve, and the other limit of rotational displacement corresponds to the second state of the valve. Advantageously the third state of the valve corresponds to one or more rotational displacements between the two maximum displacements.

A dosing unit according to the invention for an infusion pump device comprises a valve according to the invention as discussed above. Advantageously in such a dosing unit the valve is arranged to fluidly connect in the first state a cylinder of a piston pump with an outlet conduit connectable to an infusion set, and in a second state with an inlet conduit connectable to a liquid medicament reservoir.

Advantageously the valve of such a dosing unit comprises a valve seat and a valve member that are rotatable to each other. Either said valve seat or said valve member is an integral part of the cylinder. In a particular advantageous variant the rotation axis of the valve seat and the valve member is collinear to the longitudinal axis of the cylinder.

An infusion pump device according to the invention comprises valve according to the invention, and/or a dosing unit according to the invention.

In a method according to the invention for conveying liquid medicament in an infusion pump device, a pump cylinder of a dosing unit is fluidly connected to a liquid medicament reservoir, and is filled with a certain amount of liquid medicament retrieved from said reservoir. The pump cylinder is subsequently fluidly connected to an infusion site interface, and liquid medicament in the pump cylinder is pumped toward an infusion site interface, in one or more portions. Prior to connecting the cylinder to the infusion site interface, the cylinder is connected to environment.

In an advantageous variant of such a method, after pumping each single portion of the liquid medicament toward the infusion site interface, the cylinder is disconnected from the infusion site interface.

In the given description the one part of the valve remaining static in regard to the cylinder is named valve seat, while the other part of the valve, which is displaced in regard to the first part during use, is named valve member. This nomenclature, however, is only chosen as a convention and is not intended to limit the invention. Particularly in the most advantageous embodiments it is the valve seat that is rotated in regard to the structure of the infusion pump device, while the valve member remains static in regard to the structure. Thus the terms valve seat and valve member have to be understood as exchangeable.

### Brief description of the drawings

In order to facilitate a fuller understanding of the present invention, reference is now made to the appended drawings. These references should not be construed as limiting the present invention, but are intended to be exemplary only.
- Figure 1: schematically shows two infusion pump devices according to the prior art.
- Figure 2: schematically shows (a) an infusion pump device with a dosing unit according to the invention, and (b) a schematic view of the valve of the dosing unit according to the invention.
- Figure 3: shows a combined pump cylinder and valve of an advantageous embodiment of a dosing unit according to the invention, (a) in an isometric view, (b) in a longitudinal section along plane A-A, and (c) in a longitudinal section along plane B-B.
- Figure 4: shows the pump cylinder and valve seat of the embodiment in Figure 3, (a) in an isometric view, (b) in a front view onto the valve seat, and (c) in a longitudinal section along plane A-A.
- Figure 5: shows the outer component of the valve member in Figure 3, (a) in an isometric view, (b) in a longitudinal section along plane A-A, and (c) in a longitudinal section along plane B-B.
- Figure 6: shows the inner sealing component of the valve member in Figure 3, (a) in an isometric view, and (b) in a longitudinal section along plane A-A.
- Figure 7: shows different embodiments of a valve member of a dosing unit according to the invention in frontal view onto the sealing area.
- Figure 8: discloses another advantageous embodiment of a dosing unit according to the invention with radially mounted valve member, (a) in a view onto the front end of the cylinder along the longitudinal axis, (b) in a longitudinal section along plane A―A, (c) in a cross section along plane B―B, the valve being in a first state, and (d) in a longitudinal section along plane C―C, the valve being in a second state.
- Figure 9: discloses yet another advantageous embodiment of a dosing unit according to the invention, with axially mounted valve member, (a) in a view onto the front end of the cylinder along the longitudinal axis, (b) in a cross sectional view along plane A―A, (c) in a cross section along plane B―B, the valve being in state I, (d) showing a detail view, (e) in an isometric view of the valve member, (f) in a cross section through the valve member alone, along plane C―C, and (g) in a isometric view of the cylinder and the valve seat alone.
- Figure 10: schematically shows a valve similar to the variant in Figure 9, with a relief area, in which the sealing elements of the valve member are relied from mechanical stress.
- Figure 11: shows three variants of a valve member for use in a dosing unit similar to the one in Figures 9 and 10.
- Figure 12: depicts an embodiment of a valve according to the invention with three different conduits connectable to the cylinder.
- Figure 13: shows a further embodiment of a dosing unit according to the invention, with separate valve seat and cylinder, (a) in a longitudinal section, (b) in a longitudinal section along plane A-A, and (c) in an isometric view.
- Figure 14: shows a variant of a dosing unit according to the invention where an open cylinder is closed on one end by the valve member.
- Figure 15: shows an embodiment of a dosing unit with a valve member in the form of a valve disc, (a) in a longitudinal section, and (b) in a top view on the valve disc alone.
- Figure 16: shows a variant of the valve in Figure 15, (a) in a longitudinal section, and (b) in a top view on valve disc alone.
- Figure 17: schematically shows a variant of a valve according to the invention that is particularly suitable for long time storage prior to use, (a) in a side view of the valve in state I, (b) in a cross-section of Figure 17(a) along plane A-A, (c) in a side view of the valve in storage mode, and (d) in a cross-section of Figure 17(c) along plane A-A.

### Description of embodiments of the invention

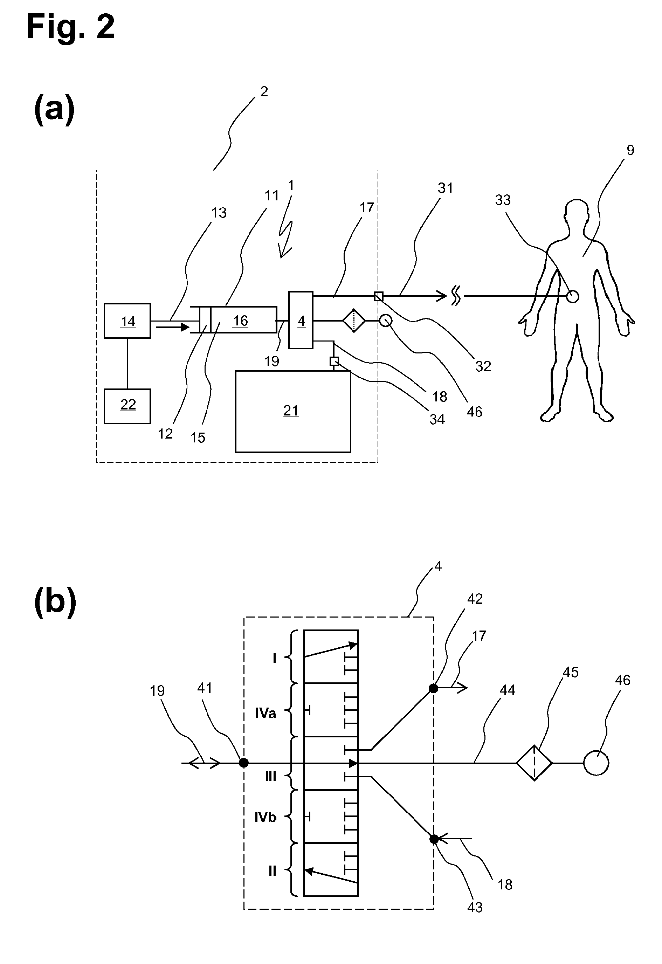

A schematic view of an embodiment of an infusion pump device according to the invention is shown in Figure 2(a). The infusion pump device 2 comprises a primary reservoir 21, a dosing unit 1, and a control unit 22. The primary reservoir holds the supply of liquid medicament, e.g. insulin solution.

Advantageously, the primary reservoir 21 is a fully or partially collapsible container, thus its content is not pressurized in regard to environmental pressure. Suitable containers for that purpose are for example known from EP 2193815 and EP 2179755 of the applicants, the disclosure of which is hereby incorporated by reference. Instead of a flexible reservoir an rigid ampoule or cartridge can be used. The preferred pressure equalization can then be achieved with a venting conduit, or a freely displaceable piston. In the latter case the piston may be subject to a biasing spring force, in order to overcome friction between piston and cartridge.

The dosing unit 1 comprises a piston pump with a pump cylinder 11, and a piston head 12 slidably arranged within the cylinder and sealingly closing the cylinder, thereby defining an inner volume 15 of the pump cylinder. The piston head 12 is actuated by a drive system 14, for example by operatively coupling a piston shaft with the drive system. Several suitable designs of such actuation means are known from the prior art. A discussion of certain possible variants is for example given in paragraph 23 in WO 2009/068251 A1. The drive system 14, and thus the dose of administered medicament, is controlled by a control unit 22 of the infusion pump device.

The dosing unit 1 can retrieve liquid medicament from the primary reservoir 21 via inlet conduit 18, and can pump the liquid medicament in small, accurate doses via outlet conduit 17, infusion tubing 31 and an infusion cannula of an injection site interface 33 into the body of the patient 9. The infusion tubing 31 is fluidly coupled to the outlet conduit 17 with a suitable coupling unit 32.

Alternatively, the infusion tubing 31 may be omitted. In such an embodiment, the whole infusion pump device is directly located at the infusion site and is attached to the body, e.g., via an adhesive pad.

The correct flow of the liquid medicament within the infusion pump device is controlled by valve 4, which is schematically shown in more detail in Figure 2(b). The shown valve 4 is a 4/5 way valve, having five distinct states I, IVa, III, IVb, II that can be switched in sequential order. Pump connection 41 of the valve is fluidly connected to the inner volume 15 of the pump cylinder 11 via passage conduit 19. Infusion site connection 42 is fluidly connected with outlet conduit 17. Reservoir connection 43 is fluidly connected to inlet conduit 18.

In active state I of the valve 4, pump connection 41 is interconnected to infusion site connection 42, thereby establishing a fluid path between the inner volume 16 of the pump cylinder 11, the passage conduit 19, the valve 4, the outlet conduit 17, and the tubing coupling 32, and from there to the infusion site interface 33. State I is applied during the pump mode of the dosing unit 1, when the piston 12 is displaced into the cylinder and liquid medicament in the cylinder is expelled through the fluid path toward the patient.

For refilling the secondary reservoir 16 of the dosing unit 1, the valve 4 is switched to active state II, where reservoir connection 43 is interconnected to pump connection 41, thereby establishing a fluid path between the primary reservoir 21 and the inner volume 1 of the pump cylinder 11, via the inlet conduit 18, the valve 4, and the passage conduit 19. The dosing unit is now in its refilling mode.

For switching the valve between state I and state II, the valve switches to an intermediate state, where all connections 41, 42, 43 are completely disconnected, to ensure that no unwanted connection can temporarily exist during the transition from one active state to another. This is a measure known from the prior art (see e.g. Figure 1 (b)). In the valve 4 in Figure 2(b) these intermediate state correspond to states IVa and IVb.

In comparison to the prior art valve in Figure 2(b), the valve 4 as used in a dosing unit 1 according to the invention has an additional active state III, to which the valve switches between the intermediate states IVa and IVb. In this state III the inner volume 16 of the cylinder 11 is fluidly interconnected to environment 46, via passage conduit 19, valve 4, and venting conduit 44. The dosing unit is in the venting phase.

This additional state III provides different advantages over the prior art. For example, the pressure differential between the inside 16 of the pump cylinder 11 and the environment 46 should be essentially zero, except when displacing the piston 12 in either of the refill mode or the pump mode, where the pressure differential conveys the liquid within the fluid system. If a user changes his location between two dosing events, for example during travel, the changing atmospheric pressure will lead to a positive or negative pressure differential between the pump cylinder and the surrounding atmosphere. In Figure 2(b) an additional sterile filter 45 is arranged in the venting conduit 44, which is, however, not necessary.

A substantive over-pressure in the cylinder may arise when filling the cylinder with the valve being in state I. If a substantive over-pressure is present in the primary reservoir, e.g. due to thermal expansion and/or mechanical pressure exerted onto the reservoir, this over-pressure would be transferred to the cylinder. When a prior art valve subsequently switches back from state I to state II via an intermediate break state, this results in the over-pressure being relieved by an undesired administration of liquid to the patient. In the worst case this can lead to a potentially hazardous overdosing event. In a dosing unit according to the invention, however, this is prevented by passing state III in between. Any pressure differential that may potentially exist between the inner volume 16 of the pump cylinder 11 and the environment will automatically be equalized through venting conduit 44, when the dosing unit is temporarily in the venting mode, and the valve is in state III. This constitutes an additional advantageous effect of the invention

Another advantage of such a design is the additional level of safety provided. If in a prior art dosing unit one component fails, for example if the drive system has a malfunction, this malfunction does not result in a potentially hazardous event, since the valve will provide a barrier in the fluid system. Only if both components fail, a potentially hazardous event can take place. A valve according to the invention now provides an additional level of safety. According to the basic principle of the invention, no fluid connection can be established due to a failure of a valve between the primary reservoir and the outlet conduit, since the liquid stream will have to pass the opening toward the venting conduit and will flow out of the venting conduit. Thus the venting conduit adds an additional level of isolation between the outlet conduit and the dosing pump and the primary reservoir.

In the purely schematic depiction of the valve 4 in Figure 2(b) the different active and intermediate states of the valve are shown as a linear shift mechanism. In a more advantageous embodiment of such a valve, the valve is switched in a rotational motion.

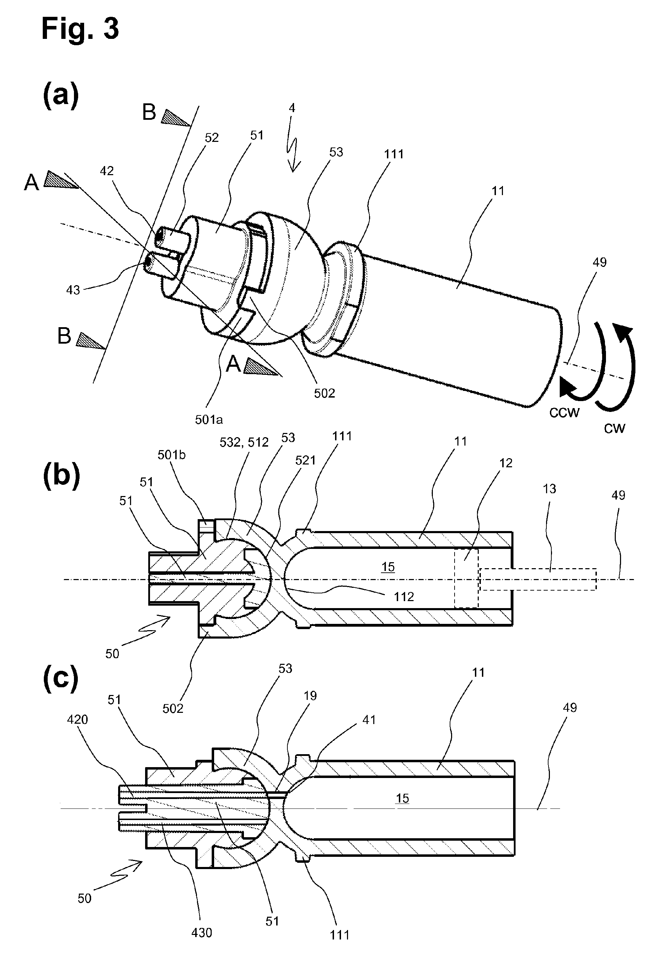

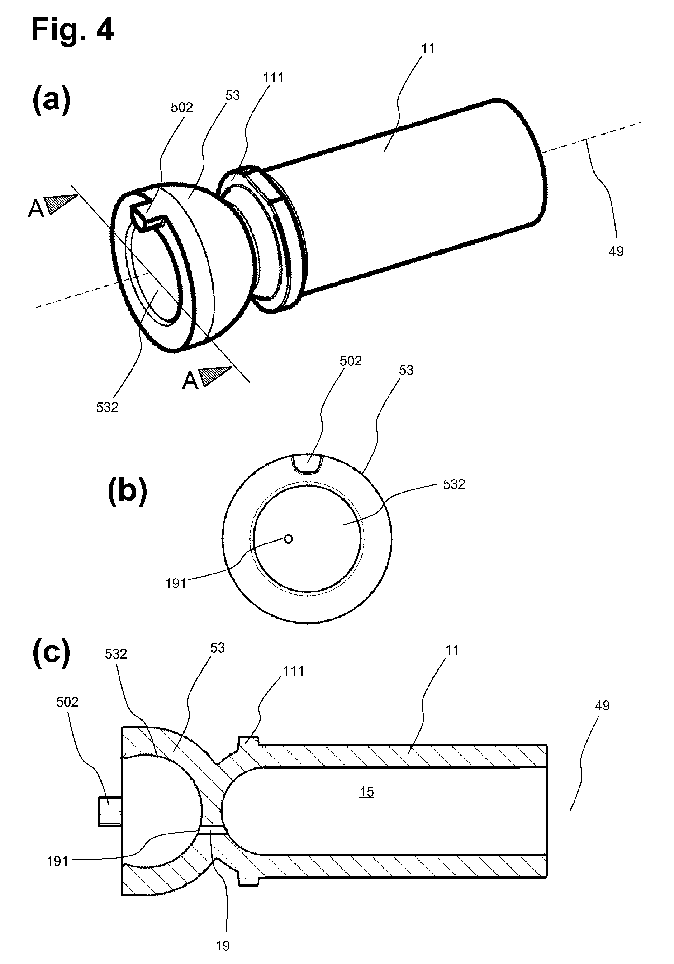

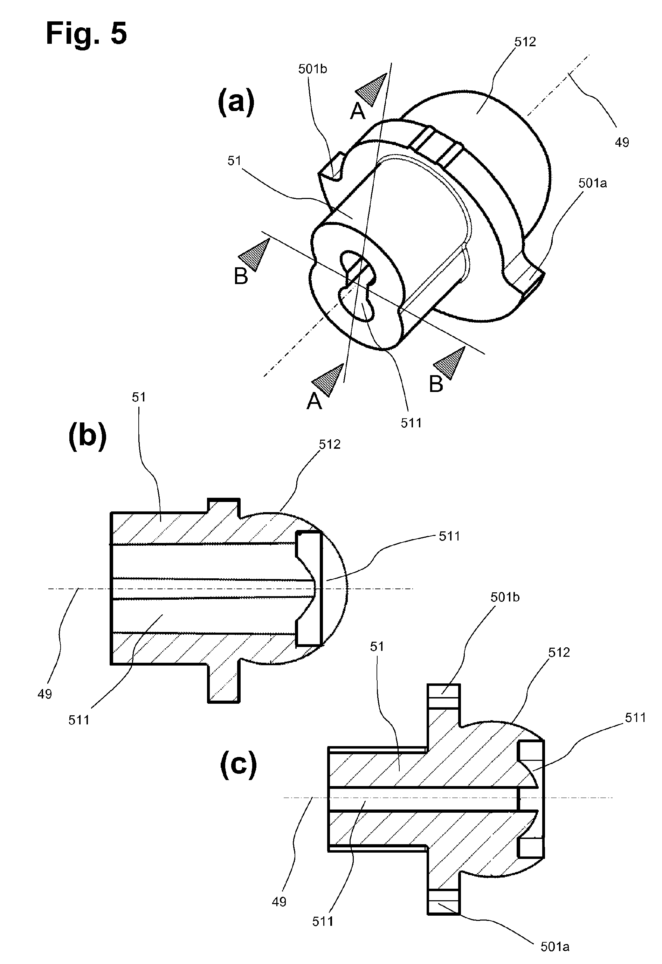

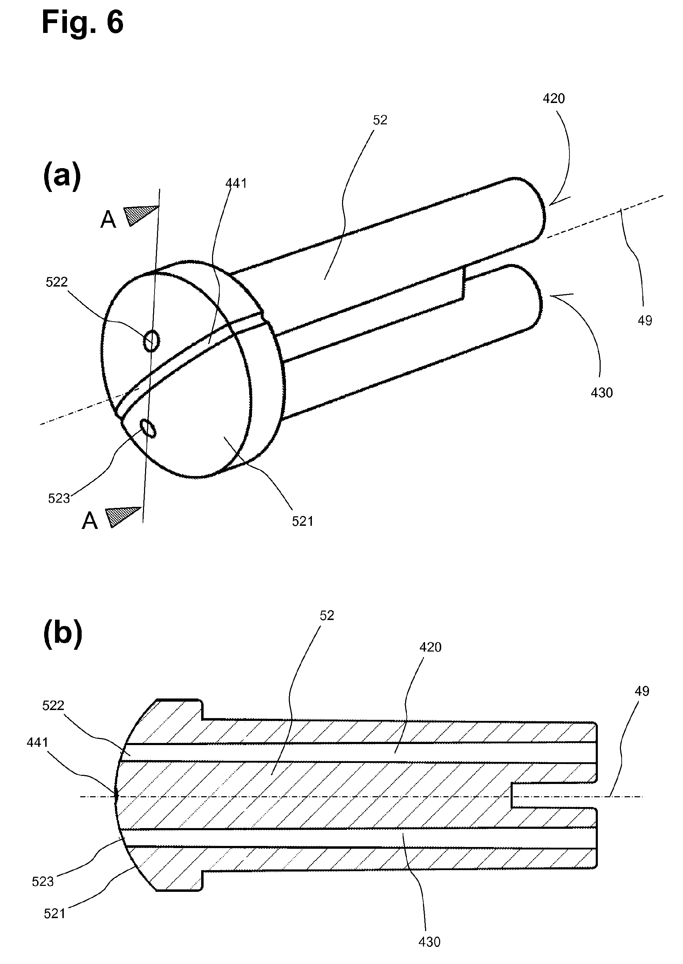

A possible embodiment of a combined pump cylinder and valve of an advantageous embodiment of a dosing unit 1 according to the invention is shown in Figures 3 to 6. While Figure 6 shows a combined pump cylinder 11 and valve 4, Figure 4 shows the pump cylinder 11 with integral valve seat 53, and Figures 3 and 5 show the two components 51, 52 of a disassembled valve member 50.

The shown embodiment of a dosing unit comprises a piston pump with a cylinder 11 and a piston 12, 13 (schematically shown with dashed lines), and a valve 4, arranged at the end of the cylinder opposite to the piston. The valve 4 comprises a valve seat 53 and a valve member 50. The valve seat 53 is realized as an integral part of the cylinder 11, which is advantageously manufactured by injection moulding. The cylinder is rotatably mounted in the dosing unit, having a guiding ring 11 mounted in a corresponding bearing structure (not shown) of the dosing unit.

A passage conduit 19 is arranged between the inner volume 15 of the cylinder 11 and a concave holding structure 532 of the valve seat 53. Said passage is offset from the longitudinal axis 49 of the cylinder and valve. Said concave structure 532 interacts with a shape-matched, spherically shaped counterpart 512 of the valve member 50, which is rotatably mounted within structure 532. Instead of the shown spherical shape of the interacting surfaces 512, 532, any other rotationally symmetric shape could be applied. The concave structure 532 is undercut, in order to positively locking the valve member 50 in a longitudinal direction in the valve seat 53. For assembly the member seat is snapped over the valve member.

The valve member 50 is rotationally fixed in regard to the longitudinal axis 49. The valve member comprises a first, outer component 51 and a second, inner component 52, which is arranged in a corresponding cavity 511 of the outer component 51. The outer component is advantageously made from a rigid polymer material, such that friction between valve seat and valve member is minimal. The inner component should provide a certain elasticity, since it acts as the sealing 521 of the valve, sealingly connecting the opening 192 of the passage conduit 19 and the openings 522, 523 of the connection conduits 420, 430.

Advantageously the valve member is manufactured by two component injection moulding. For the outer component advantageously a rigid thermoplastic polymer material used that is acceptable for medical use, e.g. Acrylnitril-Butadien-Styrene (ABS), Polyamide (PA), or Polycarbonate (PC). The same rigid materials are advantageously used for the valve seat and the cylinder. For the inner component a comparably soft thermoplastic material is used, for example thermoelastic polymers of the TPE-V (cross-linked olefins), TPE-S (styrene blockcopolymers), TPE-U (urethane) class.

The inner component 52 comprises two connection conduits 420, 430, arranged parallel to the longitudinal axis. They open toward openings 522, 523 on the sealing area 521 of the inner component 52, which forms part of the spherical surface 512 of the valve member 50. The position of the two openings 522, 523 is chosen such that the opening 192 of the passage conduit 19 overlaps with one opening 522 when the cylinder 11 is in a first rotational orientation (corresponding to valve state I), and overlaps with the other opening 523 when the cylinder is in a second angular orientation (corresponding to a valve state II). When two openings overlap, a fluid connection between the passage conduit 19 and one of the connector conduits 420, 430 is established. The other conduit 430, 420 is sealingly closed. The parameters of the material for the sealing area and the valve member, as well as the dimensions of the parts are chosen such that in the intermediate states, where the openings do not overlap, the conduits 19, 420, 430 are sealingly closed. As can be seen in Figure 6(a), a venting groove 441 is diagonally arranged across the sealing area 521. Said venting groove is fluidly connected to environment pressure, via a venting conduit (not shown).

For switching the valve between the two active states I and II, the valve member 50 and the cylinder 11 with its integral valve seat 53 have to be rotated in regard to each other, along axis 49. In the given embodiment, the cylinder 11 is rotated by 180°, while the valve member 50 is fixed. The rotation of the cylinder may be actuated similarly to the dosing unit disclosed in EP 216273 A1, and will be discussed further below.

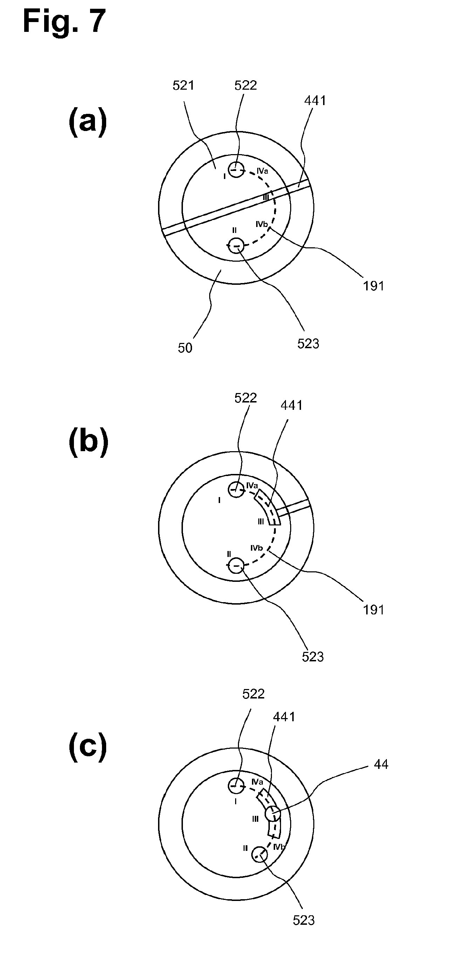

When the cylinder 11 is rotated, the opening 191 of the passage conduit travels in regard to surface 521 of the valve member on a circular path 191. Different angles of rotation, such as, e.g., 90° may be used as well. This is schematically explained in Figure 7(a), where a front view of a valve member 50 is shown. The path 191 of the opening 192 is shown as a dashed line. When the opening 192 lies on opening 522, the valve is in active state I. Upon rotation of the cylinder, the opening travels along the path, passing intermediate break state IVa, before reaching the venting groove 441. When the opening 192 overlaps with the venting groove 441, the cylinder 11 is fluidly connected with environment pressure, and the valve is in active state III. The opening 192 then passes the second intermediate break state IVb, and finally reaches the active second state II, with openings 192, 523 overlapping.

While during the pumping mode and refilling mode the valve remains a comparably long time in the two states I and II, the time period in which the valve remains in state III is comparably short and depends on the rotational motion of the cylinder 11. If a constant angular velocity of the rotation is applied, the length of the venting mode period depends on the angular velocity, the radius of the circular path 191, as well as the diameter of the opening 192 and the width of the venting groove 441. For a typical exemplary embodiment, the time for switching between state I and state II lies in the range of some seconds.

To increase the time period in which venting of the cylinder 11 can take place, the rotation of the cylinder may be slowed down, the rotation may be temporarily halted in the state III, l, or the width of the venting groove 441 may be adjusted. Such measures, however, are not necessary for typical and favourable embodiments.

In the embodiment shown in Figure 6(a), Figure 7(a), the venting groove 441 crosses the complete sealing area 521. Such a design has the advantage that the effective diameter of the venting conduit is larger, and that there exist two venting pathways. Another important advantage is the fact that in the case of a malfunction of the valve, no fluid path between primary reservoir or inlet conduit and the outlet conduit can come into existence. Any liquid unintentionally leaving the primary reservoir will drain via the venting groove 441 and the venting conduit.

For some designs, the dosing unit may be long-term stored in state III, resulting in the sealings being in a relieved, stress-free state.

Various other designs of the sealing area and the arrangement of the openings 522, 523 and the venting groove 441 are possible within the scope of the invention. Two examples are shown in Figures 7(b) and (c). In Figure 7(b) the venting groove 441 comprises a radial segment arranged on the path 191, thereby increasing the length of the venting mode period when the switching rotation takes place at a constant velocity. In Figure 7(c) the venting conduit 44 directly opens toward the sealing surface 521, and is combined with a segmental venting groove. In this particular embodiment the two openings 522 523 of the connection conduits 420, 430 are not arranged on opposite ends, but closer on the switching path 191. Thus the switching angle is smaller than 180°. Similarly the switching angle may be larger than 180°.

To switch the valve 4 between the different states, the cylinder 11 with integral valve seat 53 is rotated in regard to the fixedly mounted valve member 50. This may for example be achieved by a separate actuation means arranged to rotate the cylinder around its longitudinal axis 49. However, in order to provide a reliable and cost efficient dosing unit, the number of separate components and systems should be as small as possible. In the given embodiment the valve switching mechanism is thus combined with the piston displacement mechanism of the dosing unit (not shown). For that purpose the end of the cylinder opposite to the valve seat is provided with a threaded nut, which interacts with a threaded portion of the piston shaft. The piston shaft is operatively connected to the drive system in such a way that it can be rotated both clockwise (cw) and counterclockwise (ccw), while at the same time being displaceable along axis 49.

In Figure 3(a) to (c) the valve 4 is in state I, with the inner volume 15 being connected to conduit 420. A cam 502 of the valve seat 53 is in contact with a stopper 501 a of the valve member. Thus a further rotation of the cylinder 11 in the counterclockwise direction ccw is blocked. In this state I the dosing unit is in its pumping mode. By counterclockwisely rotating the threaded piston shaft, which is mounted in the threaded nut of the cylinder, the piston is shifted toward the front end 112 of the cylinder. The inner volume 15 is decreased and liquid medicament in the cylinder is expelled toward the patient.

If now the dosing unit shall change from pump mode to refill mode, this is achieved by simply reversing the rotation direction of the piston shaft from counterclockwise to clockwise cw. The friction between the threaded shaft and the threaded nut as well as between the cylinder (guiding ring 111, concave surface 532 of valve seat) and the corresponding fixed counterparts (guiding ring bearing, surface 512 of valve member) is balanced such that the frictional force acting between shaft and cylinder is larger than the frictional force acting between cylinder and bearing. As a result the cylinder is frictionally coupled to the shaft rotating into clockwise direction, and also rotates in direction B. The cam 502 wanders along the circumference of the valve member 50 and finally arrives at the second stopper 501 a. The cylinder cannot rotate any further, and the cylinder is frictionally decoupled from the still rotating shaft. The valve is now in state II, with the primary reservoir 21 and connection conduit 430 connected to the inner volume 15.

The dosing unit may especially be designed such that the cylinder and the piston move synchronously, that is, without any relative motion for this rotation. In this way, dosing errors that may otherwise result from a residual relative motion between plunger and cylinder during the switching are prevented. This is a considerable advantage, because the dosing errors resulting from several switching operations would otherwise sum up over the usage time of a dosing cylinder and a primary reservoir.

Besides pure friction coupling, other coupling arrangements may be applied, such as disclosed in the application EP 2163273 A1 of the applicants.

The piston shaft 13 continues to rotate into clockwise direction, which results in a displacement of the shaft and the connected piston head 12 out of the cylinder, thereby increasing the inner volume 15 and sucking liquid medicament from the primary reservoir into the cylinder.

If now the dosing unit shall change back to pumping mode, the rotation of the shaft is changed back to counterclockwise. The cylinder then turns from state II to state I, passing in between the state III, where the freshly refilled dosing cylinder is vented to environment pressure. After the cam 502 has reached the first stopper 501 a, the shaft continues to rotate counterclockwise and the piston starts to move inwards, expelling liquid medicament through conduit 420. In an exemplary embodiment, the time in the refill mode is in the range of about a minute.

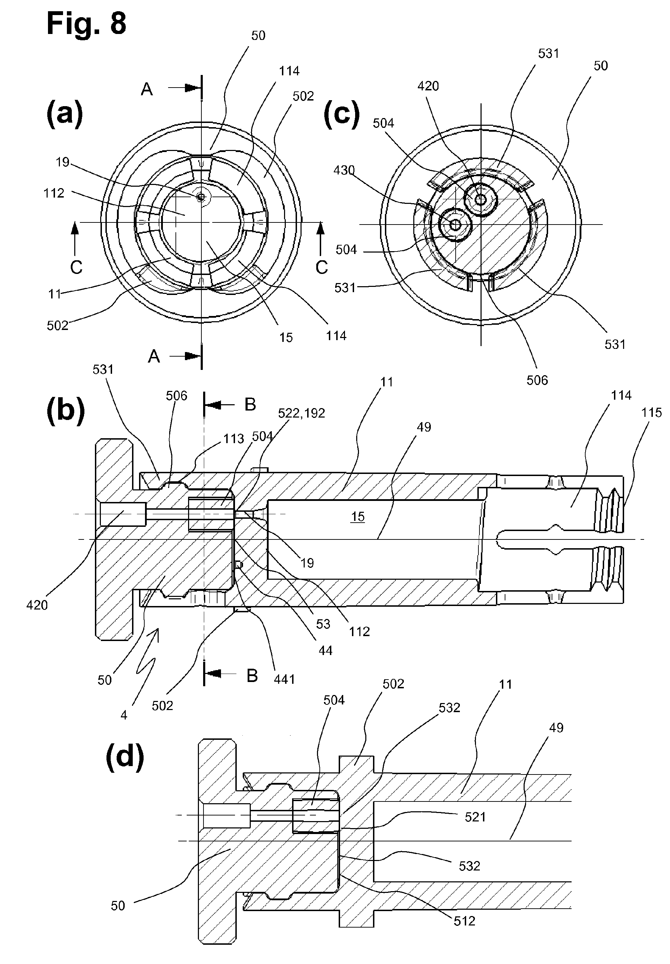

A further embodiment of a dosing unit according to the invention is disclosed in Figure 8, with a valve member 50 that is radially mounted in the valve seat 53. The valve seat 53 is an integral part of the cylinder 11, and comprises three segment-shaped snap fingers 531 with a circumferential bearing groove 113, adapted to lock onto a guiding ring 506 of the valve member 50. Thus the valve member 50 is positively locked in the longitudinal direction 49, while being freely rotatable around the axis 49. A passage conduit 19 is arranged in the front wall of the cylinder, between the cylinder front 112 and the planar front surface 532 of the valve seat 53, facing toward the valve member 50.

Valve seat, valve member and cylinder are advantageously made from a rigid polymer material as discussed further above.

At the back end of the cylinder, a threaded nut 115 is provided, adapted to interact with a threaded portion of the piston shaft (not shown). In the shown embodiment the back end of the cylinder with the threaded nut 115 is divided into four snap finger elements 114, which allows a simple assembly of the components of the piston pump.

The valve member 50 comprises two longitudinal conduits 420, 430, arranged at an angular distance of 90°. At the surface 512 facing toward the valve seat, cylindrical sealing elements 504 are arranged in the conduits 420, 430. In the assembled valve, the surfaces 512, 532 are not in contact, thereby establishing a thin venting gap 441, which is fluidly connected to environment pressure via a venting conduit 44 opening toward the valve seat surface 532, as well as the slots between the snap fingers 531.

The sealing elements 504, which are made from a suitably thermoelastomeric material as discussed further above, slightly protrude from the surface 512 of the valve member, such that in the assembled valve they are pressed against the valve seat surface 532 and seal the conduit 420, 430 against the venting gap 441.

Figure 8(b) shows the valve in state I, with the conduit 420 connected to passage conduit 19, and conduit 430 (not shown) sealingly closed. For switching the valve, the cylinder 11 with integral valve seat 53 is rotated in regard to the valve member 50 around the longitudinal axis by 90°, arriving at state II. The other conduit 430 is now fluidly connected to passage conduit 19, and conduit 420 is sealingly closed, as shown in Figure 8(d). Advantageously the rotation of the cylinder is actuated as in the previous embodiment discussed in Figures 3 to 6. To limit the 90° motion of the cylinder, two circumferential cam elements 502 are provided on the cylinder, which interact with stopper elements of the dosing unit (not shown).

In the valve discussed above in Figure 8, the intermediate valve stages IVa, IVb correspond only to the situations where the opening 192 of the passage conduit 19 is sealingly covered by the sealing rim 521 of one of the sealing elements 504.

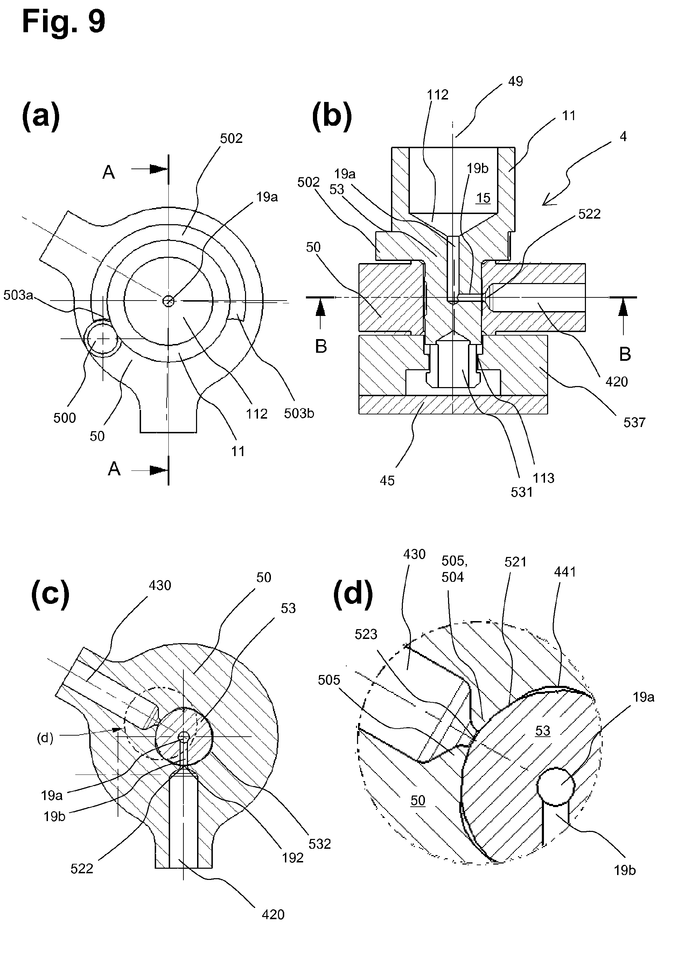

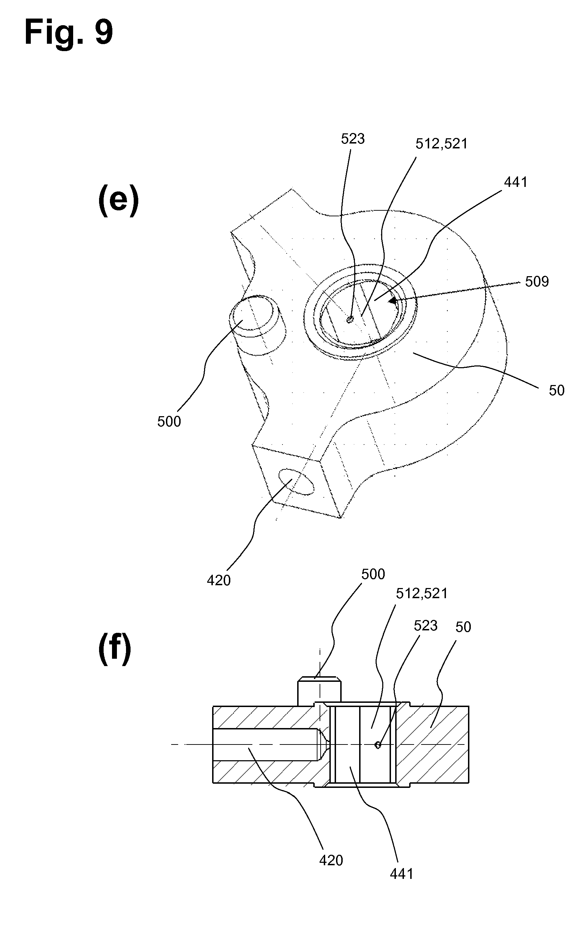

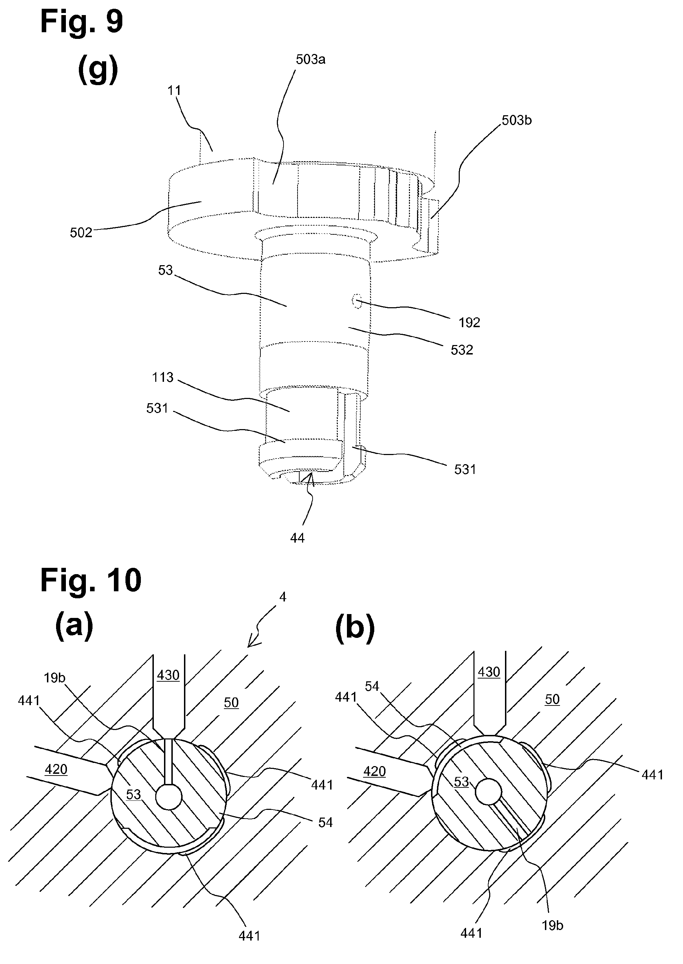

Another embodiment of a dosing unit according to the invention is shown in Figure 9. In that particular embodiment of the invention the valve 4 comprises a valve seat 53 realized as a central, cylindrically shaped valve core oriented parallel to the longitudinal axis 49. A valve member 50 is rotatably mounted on the valve seat 53. The valve seat is provided with two snap fingers 531, which lock with an opening of a locking disk 537, thereby positively locking the valve member 50 in longitudinal direction.

The valve member 50 has the shape of an oblate cylinder with two protruding arms comprising the connection conduits 420, 430, and a central cylindrical bore for the valve core 53. The conduits 420, 430 open toward the bore, at an angular distance of 120°. Arranged between the two openings 522, 523 a shallow venting recess 441 in the bore face is provided (state III of the valve), which is connected to venting conduit 44, formed by the space between the snap fingers 531 and the bore of the locking disk 537. The locking disk 537 is covered with a sterile filter 45, which protects the valve from dirt and biological contamination. For that purpose the locking disk 537 is connected with the valve seat 53 in such a way that the only connection to the environment is given by the sterile filter. Advantageously the sterile filter 45 is adapted to adsorb any liquid that may leak from the valve though the venting conduit.

In the shown embodiment a second venting recess 441 is provided in an angular position behind opening 523, the purpose of which is not to vent the cylinder 11, since opening 192 cannot reach said second venting recess 441, but to provide a barrier between conduit 420 from conduit 430 in case of a valve malfunction. Thus any liquid stream in both directions around the valve core would reach a venting recess 441 before coming close to another conduit, and would drain through that recess.

In an advantageous variant of the invention the surface of the venting recesses or grooves can be provided with a special structure and/or hygroscopic coating that improves the wetting of the surface and thus accelerates the drain of any liquid reaching the recess.

The valve seat 53 comprises a passage conduit with two portions 19a, 19b. A first portion 19a exits from the front wall 192 of the cylinder along the central axis, after a 90° turn continues as second portion 19b, and opens toward opening 192 arranged on the cylinder face. The openings 522, 523 of the conduits 420, 430 are arranged such that they can overlap with opening 192 when the valve is in the corresponding rotational position. The shown embodiment does not comprise additional sealing elements sealingly connecting the overlapping conduits 19, 420, 430, which is achieved by using comparably rigid materials for the valve seat and member, and choosing the dimensions such that a certain compression of the material takes place in the sealing areas.

As in the previously discussed embodiments the limit stop of the two states I and II is realized by cams and stoppers. A single cam 500 is provided on the valve member 50, which is advantageously fixedly mounted in the dosing unit. A segmental cam 502 of the valve seat 53 provides two stoppers 503a, 503b interacting with the cam 500 of the valve seat and delimiting the rotational motion of the rotatably mounted cylinder 11 and valve seat 53.

Figure 9 shows the valve 4 in state I, with the conduit 420 fluidly connected to the cylinder 11 via passage conduit 19a, 19b. The opening 523 of the other conduit 430 is sealingly closed by the surface 532 of the valve core, thereby disconnecting conduit 430 from the fluid system.

An advantage of the chosen design of the opening, as it is visible in Figure 9(d), is the comparably thin wall 505 surrounding the openings 522, 523. By choosing the radius of the valve core slightly larger than the radius of the bore of valve member 50, the surrounding wall 505 is compressed and acts as a circular sealing lip 504 around the opening. The sealing effect is even increased when the hydrostatic pressure in a conduit increases, since the walls 505 are pressed against the valve core surface 532 due to hydrostatic force resulting from the pressure difference. This is particularly relevant for the primary reservoir 21 connected 18 to connection conduit 430.

The continued elastic deformation of the circular wall 505 used in a valve 4 as discussed above to seal the fluid connection between conduits 19b and 420/430 can lead to creep deformation of both the wall 505 and the counter-surface 532 of the valve seat, which would be detrimental for the quality of the valve and thus advantageously should be minimized. This is particularly important if the dosing unit is intended for a long operational lifetime. But also for dosing units designed only for a comparably short life time creep should be minimized, since the shelf time prior to purchase of such a unit should be as long as possible. In an advantageous variant of the invention this goal is achieved by providing a relief recess on the valve seat. In a parking position of the valve, both sealing lips 521, which are subject to mechanical shear stress during operation, are located above the recess, where they are relieved from shear stress. Thus in this parking position no creep can take place. Advantageously such a parking position is always chosen when the dosing unit is not connected to a primary reservoir and/or an infusion set.

A possible variant of an embodiment of such a valve in a dosing unit similar to the variant discussed above is schematically shown in Figure 10, where (a) shows the valve in active state II, with conduit 430 fluidly connected to conduit 19b, and conduit 420 sealingly closed, and (b) shows the valve in the parking position, with both sealing lips 505 located over the relief recess 54, while the opening 192 is located over a venting recess 441.

The relief recess 54, which can be provided with a connection to the venting conduit, is realized as a segmental recess on the cylinder face of the valve seat 53. Since during normal operation the relief recess should not be connected to any of the conduits 420, 430, the maximum angular distance of the two conduits on the circumference of the bore is limited. In the given example the angular distance between the two conduits is slightly less than 90°, while the recess 54 spans over slightly over 90°.

An alternative approach to increase the sealing in a valve according to the invention with a cylindrical valve core is to adapt the shape of the valve core instead of the cylindrical bore. In such an embodiment the valve core 53 at the position of the opening 192 of the passage conduit may have a slightly larger radius, which will be compressed in the valve states I and II, thereby improving the sealing. To avoid creeping, a relief recess in the face of the bore can be provided, which allows to park the valve core in a position where it is not subject to mechanical stress. Instead of a dedicated relief recess a venting recess can be used.

To avoid any incorrect use of a valve unit according to the invention, advantageously means are provided that mechanically prevent the operation of the valve while in the parking position.

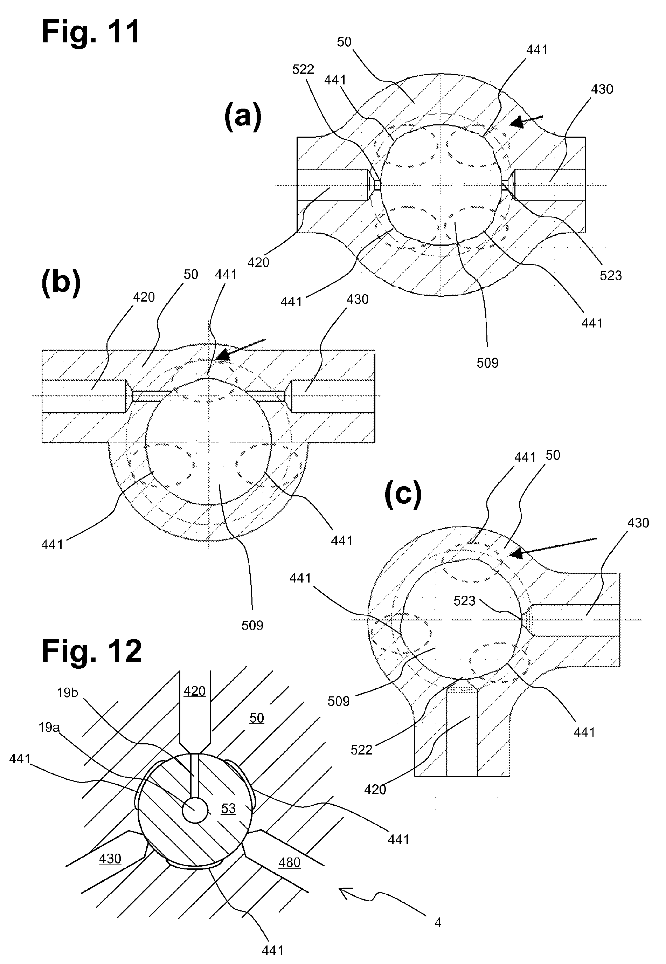

Further variants of possible designs of valve members are shown in Figure 11. In Figure 11 (a), the openings 522, 523 are arranged opposite to each other, and four venting recesses 441 are arranged on the bore surface, while Figure 11 (c) depicts a variant with rectangularly arranged conduits. In Figure 11 (c) the coaxial conduits 420, 430 are offset from the rotation center.

Figure 12 depicts a particularly useful embodiment of a valve 4 according to the invention, with three different conduits 420, 430, 480 connectable to the cylinder 11. While the functional principle of such a valve is essentially the same, it provides the considerable advantage of an additional connection, either to an additional primary reservoir, or to an additional infusion set. Particularly the first variant is very advantageous, since such a dosing unit would for example allow to easily administering different liquid medicaments (for example two different insulin products with a fast and a slow effectiveness profile). It is also possible to use two primary reservoirs with the same medicament, which then would allow the exchange of one primary reservoir or the refilling of that reservoir even in the refilling mode.

A further advantageous application of such a valve according to the invention is the possibility to use the dosing pump for refilling the primary reservoir from an external reservoir, for example a standard vial containing liquid medicament, or a cartridge intended to be used in another type of device. In such a case the external reservoir would be fluidly connected to the reservoir conduit 480, and the liquid medicament would be conveyed toward the primary reservoir by repeated actuation of the pump cylinder. Such a possibility would also allow realizing a disposable unit including a dosing unit and an initially empty primary reservoir, without the need of providing a separate mechanism for filling/refilling the reservoir. Such a disposable unit can be used together with a reusable unit comprising all the other elements of the infusion pump device that do not come into contact with liquid medicament, and thus can have a longer operational lifetime.

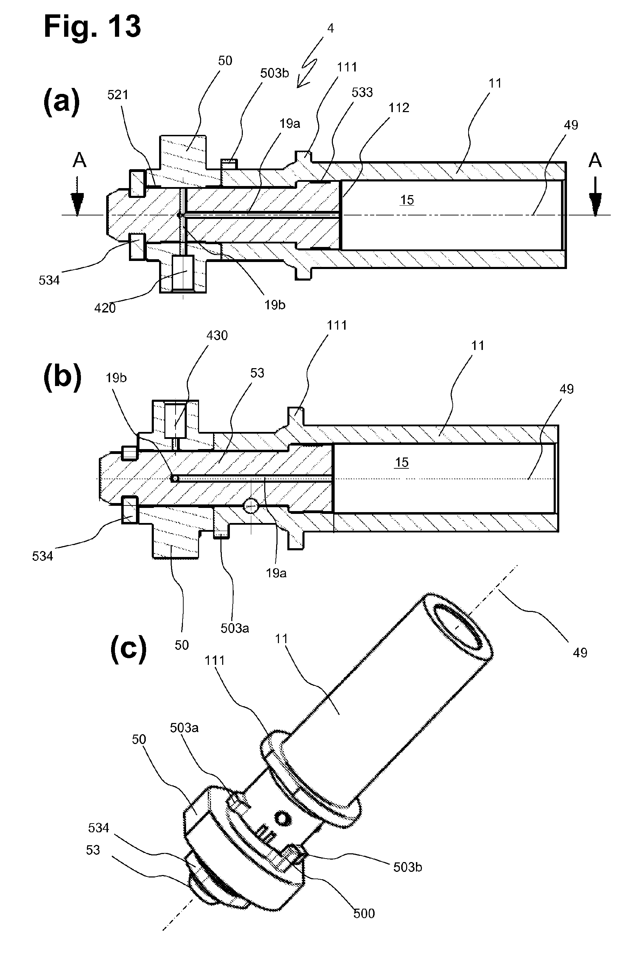

Yet another embodiment of a dosing unit according to the invention is depicted in Figure 13, comprising a cylinder 11, a valve seat 53, a valve member 50, and a locking disk 534. In contrast to the embodiments discussed so far the valve seat 53 is not an integral part of the cylinder 11, but is arranged within the cylinder. This particular variant of the invention can be assembled very easily, by introducing valve seat 53 from the back end of the cylinder, mounting the valve member 50, and finally the locking disk 534, thereby positively locking the four components in longitudinal direction. The valve seat 53 sealingly closes the front end of the cylinder with a circumferential sealing means 533, which at the same time frictionally locks valve seat and cylinder.

The valve seat with attached cylinder and the valve member are rotatable to each other around axis 49. The valve seat is provided with two stoppers 503a, 503b, which interact with a cam 500 of the valve member, thereby limiting the rotational motion between states I and II.

The valve seat 53 comprises a passage conduit with a first portion 19a along the longitudinal axis and a perpendicular second portion 19b crossing the valve seat/core. The valve member 50 comprises two conduits 420, 430 arranged perpendicular to the longitudinal axis 49. The function of the valve as such is similar to the embodiment in Figure 9, with the faces of the valve core 53 and the bore of the valve member 50 sealingly interacting with each other. Figure 13(a) and (b) shows the valve in state I, with passage conduit 19b fluidly connected to conduit 420, while conduit 430 is sealingly closed by the valve core.

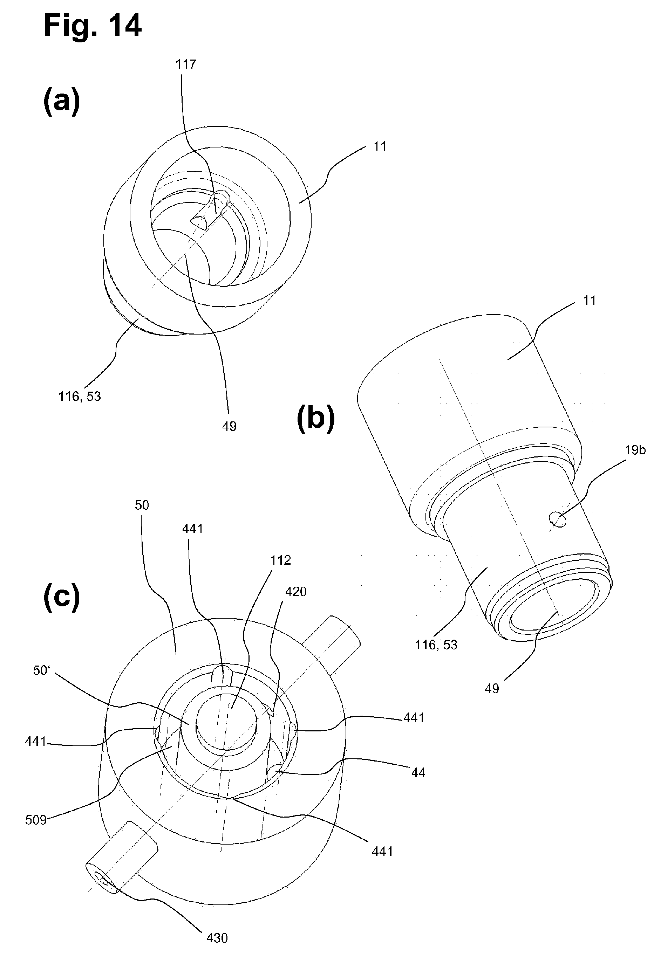

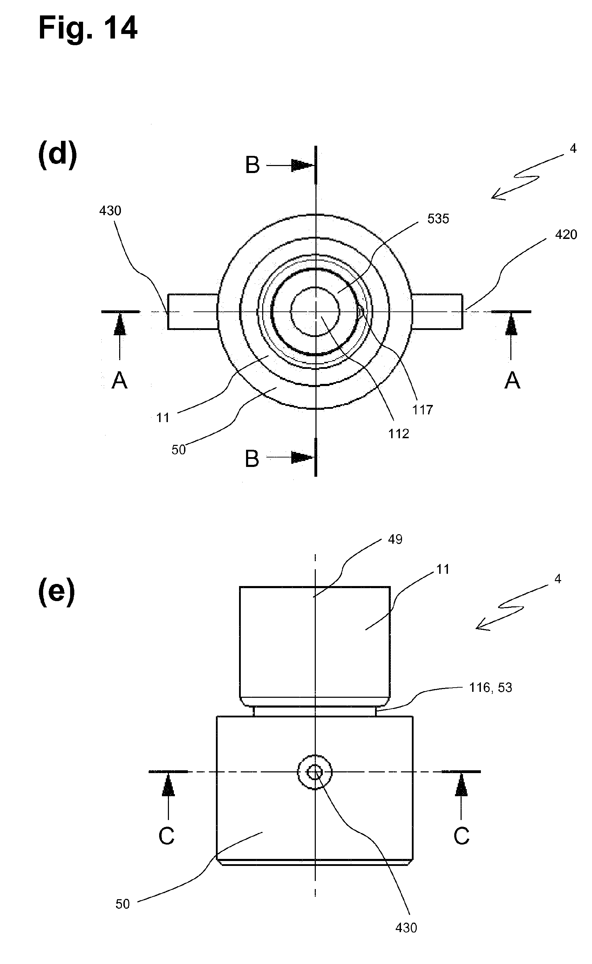

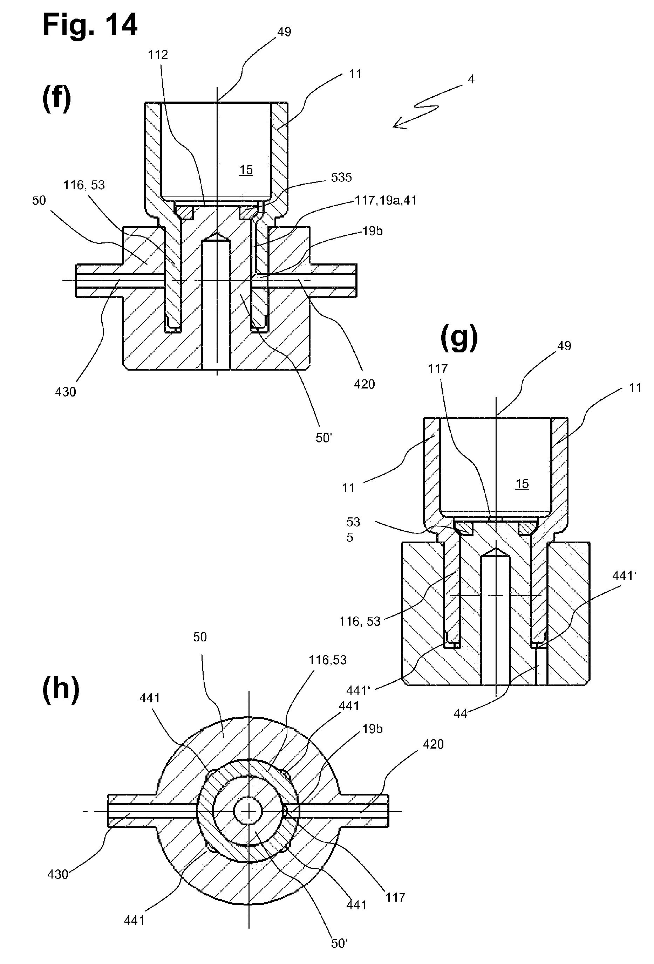

Another valve according to the invention is disclosed in Figures 14(a) to (h). Figures 14(a) and (b) show isometric views on the valve seat 53 / cylinder 11, while Figure 14(c) shows the valve member 50. Figures 14(d) to (h) show the assembled valve 4, (d) in a top view along longitudinal axis 49 with view onto the cylinder bottom 112, (e) in a side view with view onto one of the connection conduit tubes, (f) in a cross-section along plane A-A, (g) in a cross-section along plane B―B, and (h) in a cross-section along plane C―C.

A hollow cylinder 11 comprises at its front end a constricted portion 116 that acts as the valve seat 53, which is rotatably mounted in a cylindrical bore 509 of the body of valve member 50, 50'. A circumferential shoulder of the cylinder rests on the body of valve member 50. A cylindrically shaped inner portion 50' of the valve member is arranged within the cylinder. A locking ring 535 is assembled, e.g. by ultrasonic welding, to the inner portion 50' of the valve member, and determines the relative axial position of valve member and cylinder 11. In addition, the locking ring 535 axially secures the cylinder 11 with respect to valve member 50. The locking ring 535 can alternatively be assembled to cylinder 11, or can be replaced by a different means, such as a snap ring.

In the shown embodiment, the liquid-tight connection between cylinder wall and cylinder bottom is provided along the abutting longitudinal surfaces of the inner part 50' of the valve member and the inner side of the valve seat 53.

The restricted portion 116 / valve seat 53 of the cylinder comprises a longitudinal groove 117 arranged on the inner cylindrical surface of the restricted portion, which together with the outer surface of the cylindrical inner portion 50' of the valve member sealingly forms a passage conduit portion 19a opening toward the inner volume 15 of the pump cylinder. Two conduits 420, 430 are radially arranged in the portion 50 of the valve member on the other side of the cylinder wall. A radial bore 19b in the wall of the valve seat 53 is provided to establish a fluid connection between passage conduit 19a / groove 117 and one of the connection conduits 420, 430, when the opening of one of the connection conduits and the bore 19b overlap, depending on the rotational orientation of valve seat and valve member to each other.

Four longitudinal grooves 441 are arranged in the cylinder wall of valve member 50, which are connected via a small circumferential gap 441' between the restricted portion 116 and the bottom of bore 509 to a venting conduit 44.

The valve in Figures 14(d) to (h) is in state I, with the passage conduit 117, 19a, 19b being fluidly connected to the infusion site connection conduit 420. When now the valve is switched to state II, clock-wise or counter-clock-wise, depending on the configuration chosen for the valve, the passage conduit 19a, 117, 19b, which acts as the pump connection 41, is temporarily connected to environment (state III) whenever the horizontal bore 19b passes a groove 441. In the given embodiment the valve thus passes two states III when switching from state I to state II, and back.

Following the main principle of the current invention, there exists no fluid path between the infusion site connection conduit 420 and the reservoir connection conduit 430 that does not pass at least one of the grooves 441, 441'. Thus under no circumstances liquid can flow from the reservoir through the valve 4 to the infusion site, since it would drain through grooves 441, 441' toward the venting conduit 44.

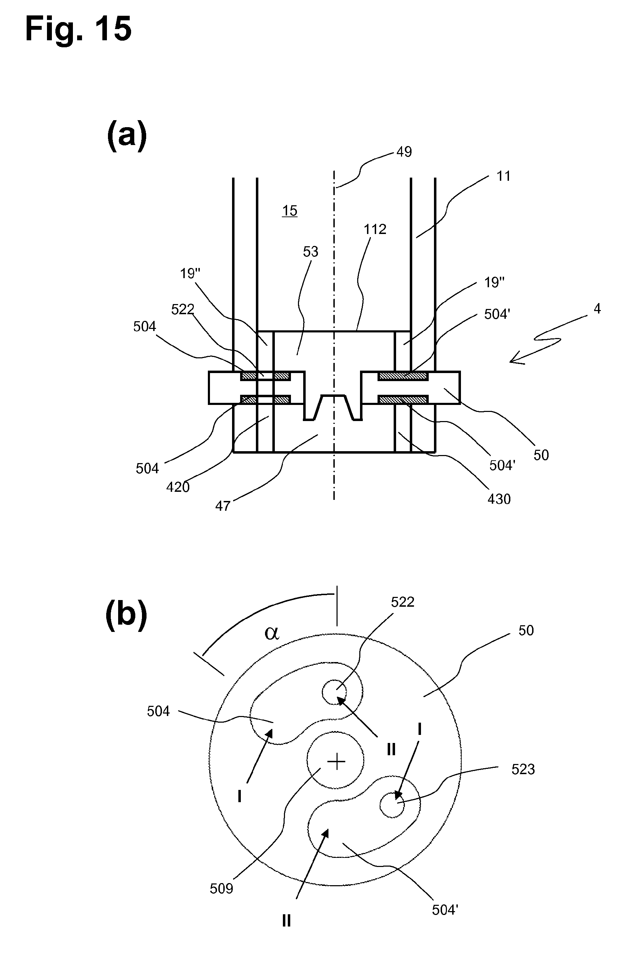

An embodiment of a dosing unit with a valve member in the form of a valve disc is shown in Figure 15. A cylinder 11 with an integral valve seat 53 is connected to bearing 47. A disk shaped valve member 50 is rotatably mounted between valve seat and bearing. The valve seat and bearing are connected for example by a snap lock mechanism, or by ultrasonic welding. The valve seat comprises to axial passage conduits. One passage conduit 19' is collinear with a infusion site connection conduit 420 arranged in the bearing body, and the second passage conduit 19" is collinear with an reservoir connection conduit 430.

The valve disk 50 comprises two sealing elements 504, 504' made from a elastic polymer material as already discussed further above. Two bores 522, 523 are located in the sealing elements, such that in a state I of the valve the bore 522 in sealing element 504 fluidly connects one passage 19' with the conduit 420, and the other sealing element 504' disconnects the other passage 19" from the conduit 430. To switch the valve to state II, the cylinder 11 and valve seat 53 are rotated in regard to the disk by an acute angle α, disconnecting the first conduit 420 from the cylinder and connecting the second passage 19" to the second conduit 430.

The venting state III can be realized for example with grooves arranged on the sealing elements that are fluidly connected to a venting conduit, similar for example to the embodiment shown in Figures 3 to 6.

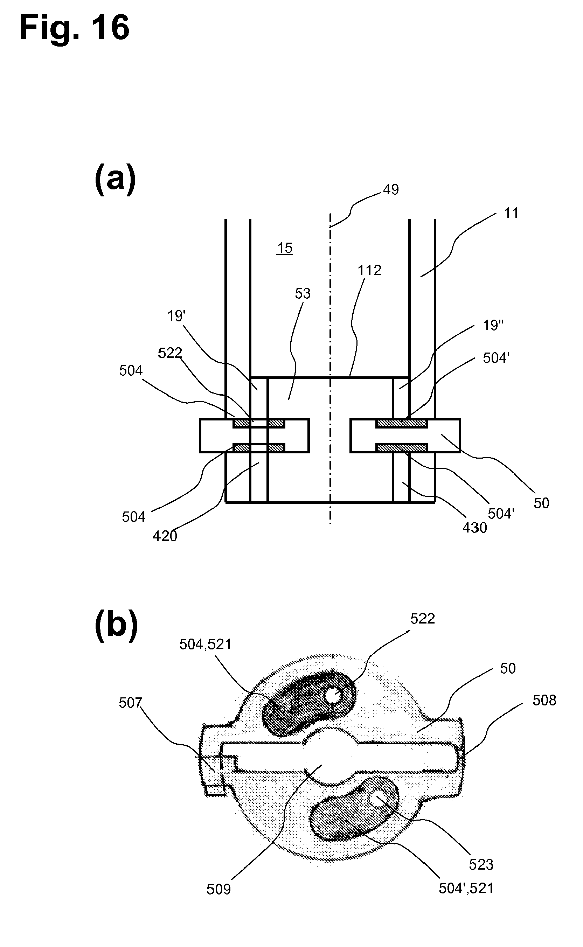

Figure 16 depicts a variant of the valve in Figure 15(a), with no separate bearing 47. To mount the valve disk in such a valve, the valve disk of Figure 16(b) is particularly suitable. The two half portions of the disk are pivoted around hinge 508, for embracing the valve seat, and are locked with a suitable mechanism 507 in the final conformation.

The present invention is not to be limited in scope by the specific embodiments described herein. Indeed, various modifications of the present invention, in addition to those described herein, will be apparent to those skilled in the art from the foregoing description and accompanying drawings. Thus, such modifications are intended to fall within the scope of the appended claims. Additionally, the disclosure of any reference cited throughout the specification is incorporated herein by reference in its entirety.

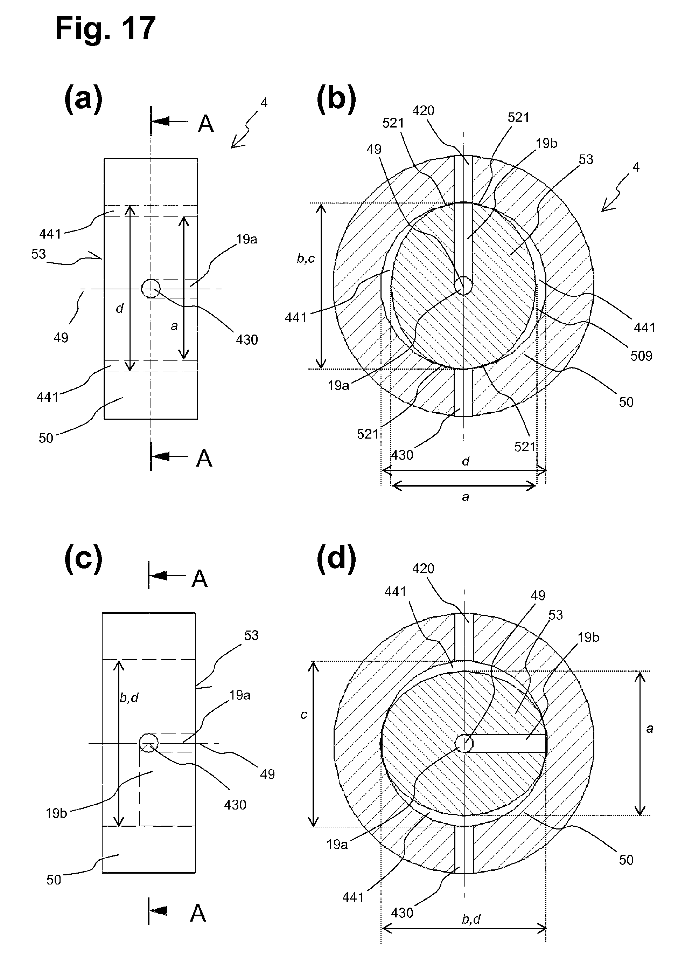

A variant of a valve according to the invention that is particularly suitable for long time storage stability prior to use is schematically depicted in Figure 17. The basic design of this particular valve in regard to the arrangement of the connection conduits 420, 430 and the passage conduit 19 is similar to Figures 9(c) and 11. The valve 4 comprises a valve seat realized as an oval-shaped, central valve core 53 oriented parallel to the longitudinal axis 49. A valve member 50 is rotatably mounted on the valve seat 53. Means for bearing the components 50, 53 and/or restricting their motions in regard to each other, which have been discussed in different variants for other embodiments, are not shown for simplicity.

The valve member 50 has the shape of an oblate cylinder comprising the connection conduits 420, 430, and a central bore 509 for the valve core 53. The conduits 420, 430 open toward the bore, at an angular distance of 180°. The central bore 509 has the shape of an ellipse with minor diameter c and major diameter d. Also the valve core 53 has the shape of an ellipse, with a minor diameter a and a major diameter b. A longitudinally oriented, first portion 19a of the passage conduit within the valve core 53 is connected to the reservoir (not shown). A second portion 19b is radially oriented along the major axis of the valve core 53.

The particular additional advantage of the shown embodiment of a valve according to the invention is related to the ratios of different diameters a, *b,* c, *d* to each other, as will now be explained in more detail.

To avoid functional failure of the valve 4 due to material fatigue of the sealing elements of the valve during storage prior to use, after manufacture the valve 4 is provided in a storage mode, which is shown in Figures 17(c) and (d). In this storage mode the second portion 19b of the passage conduit is oriented in an angle of 90° to the connection conduits 420, 430. The major diameter d of the bore 509 of the valve member 50 is chosen equal or larger than the major diameter b of the valve core 53. Similarly the minor diameter c of the bore 509 is chosen equal or larger than the minor diameter a of the valve core 53. As a result, in the storage mode no portion of the valve elements 50, 53 that will later interact with each other, in order to establishing a sealing connection between passage conduit and connection conduits, is mechanically stressed. Thus no material fatigue resulting from, for example, plastic flow can take place, independently from the shelf storage time of the valve prior to first use.

During normal operation, the valve 4 can switch between state I and II, passing between one or two times a state III. In Figures 17(a) and (b) the valve is in state I, with the passage conduit portion 19b being aligned to the inlet connection conduit 420, and their openings overlapping. The major diameter b of the valve core 53 is chosen slightly larger than the minor diameter c of the bore 509. This leads to a certain compression of the elastomeric material on the contacting surfaces of the bore 509 and the valve core 53, resulting in a sealing area 521 around the connection conduits 420, 430. The exact values for b and c depend on the materials used for the valve elements. The connection conduit 420 is sealingly connected to passage conduit 19b, 19a, and connection conduit 430 is sealingly closed.

The gap 441 between the minor axis a of the valve core 53 and the wall of the bore 509 is fluidly connected to environment. Thus the gap acts as the venting conduit 44 of the valve. Any possible fluid path between the two connection conduits 420, 430 would have to pass at least one of these gaps 441. Thus any liquid that may leak from the reservoir conduit 430 in case of a malfunction of the valve due to, e.g., a leaking sealing, will drain through gap 441, and cannot reach connection conduit 420.

State II (not shown) is identical to state I, except for the orientation of the valve core 53, which is rotated by 180°, so that the passage conduit portion 19b is aligned with connection conduit 430.

To switch the valve 4 from state I to state II, the valve core 53 is rotated clock-wise (or counter-clock-wise) around axis 49. During this rotation, the gaps 441 also rotate clockwise (or counter-clock-wise) and come into contact with the connection conduits 420, 430, thereby temporarily connecting the connection conduits to environment. Figures 17(c) and (d) shows the valve 4 halfway between state I and II, with a rotation angle of the valve core of 90°.

In case the major axis d of the bore is chosen to be larger than the major axis b of the valve core, the passage conduit 19b is connected to environment latest when reaching a rotation angle of 90°.

In case the major axis b of the valve core is chosen to be the same as the major axis d of the bore, the passage conduit 19b would be not connected to environment during the switching process. In such a case, the bore wall between the two connection conduit openings can be provided for example with a segmental groove that fluidly connects the passage conduit 19b in the position of Figure 17(d) with the venting gaps 441.

Summarized one can say that for an advantageous valve 4 according to the invention as described in Figure 17, the relations have to be as follows: b>c (sealing connection in state I, II); b<d (storage mode); a<c (connection conduits connected to environment). In case b=d, an additional venting path has to be provided for the passage conduit.

### List of Reference Numerals

- 1: dosing unit
- 11: pump cylinder
- 111: guide ring
- 112: frontal end of cylinder
- 113: bearing groove
- 114: snap element
- 115: threaded nut segment
- 116: constricted portion
- 117: groove
- 12: piston head
- 13: piston shaft
- 14: drive system
- 15: inner volume of the pump cylinder
- 16: secondary reservoir
- 17: outlet conduit
- 18: inlet conduit
- 19, 19', 19": passage conduit
- 19a, 19b: portion of passage conduit
- 191: path of the opening of the passage conduit on the sealing area
- 192: opening of passage conduit
- 2: infusion pump device
- 21: liquid medicament reservoir, primary reservoir
- 22: control unit
- 31: infusion tubing
- 32: tubing coupling
- 33: infusion site interface
- 34: reservoir coupling
- 4: valve
- 41: pump connection, connection to pump cylinder
- 42: infusion site connection, connection to infusion set
- 420: connection conduit
- 43: reservoir conduit, connection to primary reservoir
- 430: connection conduit
- 44: venting conduit
- 441, 441': venting groove, venting recess
- 45: sterile filter
- 46: environment
- 47: bearing
- 480: additional connection conduit
- 49: longitudinal axis
- 50, 50': valve member
- 500: cam of valve member
- 501a a: first stopper of valve member
- 501 b: second stopper of valve member
- 502: cam of valve seat
- 503a: first stopper of valve seat
- 503b: second stopper of valve seat
- 504, 504': sealing element
- 505: surrounding wall
- 506: guiding ring
- 507: snap lock
- 508: hinge
- 509: bore
- 51: outer component of valve member
- 511: cavity for inner part of valve member
- 512: valve member surface
- 52: inner component of valve member
- 521: sealing area
- 522: opening for connection conduit 420
- 523: opening for connection conduit 430
- 53: valve seat
- 531: snap element
- 532: valve seat surface
- 533: sealing
- 534: locking disk
- 535: locking ring
- 54: idle position
- 9: patient

## Claims

1. A valve (4) for an infusion pump device (2), comprising a pump connection (41) intended to be fluidly connected to a piston pump (11, 12, 13), a infusion site connection (42) intended to be fluidly connected to an infusion set (31, 33), and a reservoir connection (43) intended to be fluidly connected to a liquid medicament reservoir (21); wherein the valve (4) can be in a first state (I), where the pump connection (41) is fluidly connected to the infusion site connection (42), and the reservoir connection (43) is sealingly closed, and in a second state (II), where the pump connection (41) is fluidly connected to the reservoir connection (43), and the infusion site connection (42) is sealingly closed, **characterized in that** the valve (4) comprises one or more conduits, grooves, and/or recesses (441) connected to environment (46) that are arranged in such a way that no path exists within the valve between the infusion site connection (42) and the reservoir connection (43), independently if such a path is closed during normal operation of the valve (4), that does not cross at least one of said conduits, grooves, and/or recesses (441), thereby establishing a drain to environment (46).

2. The valve according to claim 1, **characterized in that** the valve (4) can be in a third state (111), where the pump connection (41) is fluidly connected to environment (46), and the other connections (42, 43) are sealingly closed, whereby the valve (4) is designed in such a way that the valve has to switch to the third state (III) before it can switch to the first state (I) and/or the second state (II).

3. The valve according to claim 2, **characterized in that** the one or more conduits, grooves, and/or recesses (441) of the valve (4) serve as fluidic connection to environment (46) in state (III).

4. The valve according to any of the preceding claims, **characterized in that** the valve (4) is designed such that during switching between the different states (I, II, III) the valve goes to an intermediary state (IVa, IVb), where all connections (41, 42, 43) are disconnected.

5. The valve according to any of the preceding claims, **characterized in that** the valve (4) comprises a valve seat (53) and a valve member (50) that are rotatable to each other.

6. The valve according to claim 5, **characterized in that** the valve seat (53) comprises at least one passage conduit (19,19',19", 19a, 19b) that is fluidly connected to the pump connection (41), and the valve member (50) comprises a conduit (420) fluidly connected to the infusion site connection (42) and a conduit (430) fluidly connected to the reservoir connection (43), wherein the fluid connection between a passage conduit and the connection conduits (42, 43) is established when an opening (192) of the passage conduit overlaps with an opening (522, 523) of a connection conduit (42, 43).

7. The valve according to claims 5 or 6, **characterized by** one or more venting conduits (44) fluidly connectable (441) to a passage conduit (19, 19', 19", 19a, 19b).

8. The valve according to any of claims 5 to 7, **characterized by** means (500, 501 a, 501 b, 502, 503a, 503b) provided on the valve seat (53) and the valve member (50) that limit the rotational displacement in regard to each other, wherein one limit of rotational displacement corresponds to the first state (I) of the valve (4), and the other limit of rotational displacement corresponds to the second state (II) of the valve.

9. The valve according to claim 8, **characterized in that** the third state (III) of the valve (4) corresponds to one or more rotational displacements between the two maximum displacements.

10. A dosing unit (1) for an infusion pump device (2), **characterized by** a valve (4) according to any of claims 1 to 9.

11. The dosing unit according to claim 10, **characterized in that** the valve (4) is arranged to fluidly connect (41, 19) in the first state (I) a cylinder (11) of a piston pump of the dosing unit (1) with an outlet conduit (17) connectable (32) to an infusion set (31, 33), and in a second state (II) with an inlet conduit (18) connectable (34) to a liquid medicament reservoir (21).

12. The dosing unit according to claim 10 or 11, **characterized in that** the valve (4) comprises a valve seat (53) and a valve member (50) that are rotatable to each other, and that either said valve seat (53) or said valve member (50) is an integral part of the cylinder (11).

13. The dosing unit according to claim 12, **characterized in that** the rotation axis of the valve seat (53) and the valve member (50) is collinear to the longitudinal axis (49) of the cylinder.

14. An infusion pump device (2) **characterized by** a valve (4) according to any of claims 1 to 8 and/or a dosing unit (1) according to any of claims 9 to 12.
